# EUROPÄISCHE PATENTSCHRIFT

(11) **EP 0 648 770 B1**
(45) Veröffentlichungstag und Bekanntmachung des Hinweises auf die Patenterteilung: **17.05.2000**
(21) Anmeldenummer: 94810580.4
(22) Anmeldetag: 04.10.1994
(51) Int. Cl.: C07D 487/04, C08K 5/3415, C09B 57/00, G03G 5/06, C09K 11/06

(54) **Pyrrolo[3,4-c]pyrrole**
Pyrrolo[3,4-c]pyrroles
Pyrrolo[3,4-c]pyrroles

(30) Priorität: 13.10.1993 CH 307993; 29.06.1994 CH 207494; 29.06.1994 CH 207594; 29.06.1994 CH 207694
(43) Veröffentlichungstag der Anmeldung: 19.04.1995
(73) Patentinhaber: Ciba Specialty Chemicals Holding Inc., 4057 Basel (CH)
(72) Erfinder: Zambounis, John S., Dr., CH-3280 Murten (CH); Hao, Zhimin, Dr., CH-1723 Marly (CH); Iqbal, Abul, Dr., CH-1732 Arconciel (CH)

(56) Entgegenhaltungen:
- EP-A- 0 133 156
- EP-A- 0 224 445
- EP-A- 0 353 184
- EP-A- 0 499 011
- EP-A- 0 612 747
- US-A- 4 415 685
- Industrial Organic Pigments, VCH 1993, Seiten 1, 2 und 552
- Protective Groups in Organic Synthesis, Wiley 1991, Seiten 387-388

## Beschreibung

Die vorliegende Erfindung betrifft neue Carbamatgruppen enthaltende Pyrrolo[3,4-c]pyrrole, ihre Herstellung und ihre Verwendung als Fluoreszenzfarbstoffe, sowie als Pigmentvorstufen, die sich leicht in die entsprechenden Diketopyrrolopyrrol-Pigmente umwandeln lassen.

Aus den Patenten US-A-4 585 878 (entsprechend EP-A-0 133 156) und US-A-4 791 204 sind N-substituierte Pyrrolo[3,4-c]pyrrole bekannt. Aus der generischen Definition aller Substituenten kann entnommen werden, dass die N-Substituenten unter anderem auch Alkoxycarbonylgruppen sein können, die im US-Patent 4 791 204 beispielsweise als Methoxycarbonyl, Ethoxycarbonyl, n-Propoxycarbonyl, n-Butoxycarbonyl und n-Hexyloxycarbonyl ausgedeutet werden. Spezifische Pyrrolo[3,4-c]pyrrole mit Carbamatgruppen sind nicht beschrieben. Aus US-A-4 585 878 ist bekannt, dass die darin beschriebenen N-substituierten Pyrrolo[3,4-c]pyrrole in gelöster Form in Polymeren eine hohe Fluoreszenz zeigen.

Protective groups in organic Synthesis (Wiley 1991), Seiten 387-388, beschreibt die chemische und thermische Abspaltung von Carbamatgruppen zu Aminen.

Gemäss der vorliegenden Erfindung sind nun neue Carbamatgruppen enthaltende Pyrrolo[3,4-c]pyrrole gefunden worden, die überraschenderweise eine sehr hohe Festkörperfluoreszenz, speziell im UV, aufweisen, sich aber auch leicht, bei gleichzeitiger Verschiebung des Absorptionsspektrums, in die entsprechenden Diketopyrrolopyrrol-Pigmente umwandeln lassen und demnach den Weg zu unverhofften Applikationen frei machen. Darunter auch Pyrrolo[3,4-c]pyrrole mit N-Alkoxycarbonylgruppen deren Alkylreste an dem am Sauerstoff gebundenen Kohlenstoff verzweigt sind.

Die vorliegende Erfindung betrifft demnach Pyrrolo[3,4-c]pyrrole der Formel Pyrrolo[3,4-c]pyrrole der Formel 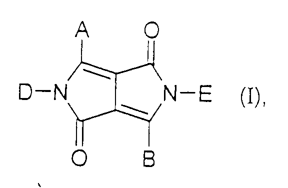 worin A und B unabhängig voneinander für eine Gruppe der Formel 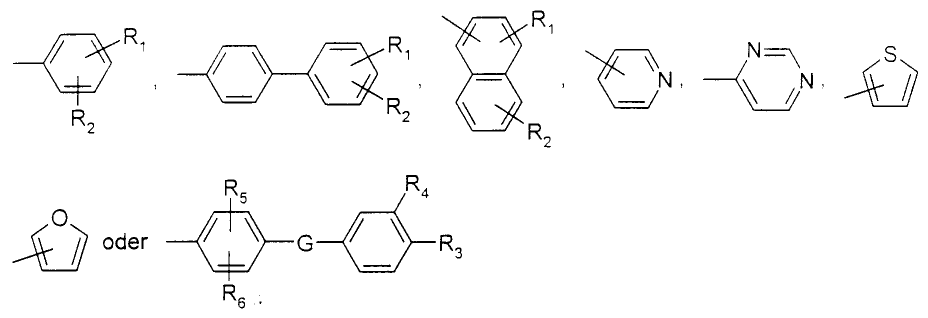 stehen, worin
R₁ und R₂ unabhängig voneinander Wasserstoff, Halogen, C₁-C₁₈-Alkyl, C₁-C₁₈-Alkoxy, C₁-C₁₈-Alkylmercapto, C₁-C₁₈-Alkylamino, -CN, -NO₂, -Phenyl, Trifluormethyl, C₅-C₆-Cycloalkyl, -C=N-(C₁-C₁₈-Alkyl), 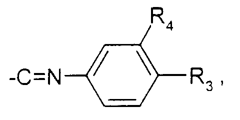 Imidazolyl, Pyrrazolyl, Triazolyl, Piperazinyl, Pyrrolyl, Oxazolyl, Benzoxazolyl, Benzthiazolyl, Benzimidazolyl, Morpholinyl, Piperidinyl oder Pyrrolidinyl bedeuten,
G -CH₂-, -CH(CH₃)-, -C(CH₃)₂-, -CH=N-, -N=N-, -O-, -S-, -SO-, -SO₂- oder -NR₇- ist,
R₃ und R₄ unabhängig voneinander Wasserstoff, Halogen, C₁-C₆-Alkyl, C₁-C₁₈Alkoxy oder -CN sind, R₅ und R₆ unabhängig voneinander Wasserstoff, Halogen oder C₁-C₆-Alkyl und R₇ Wasserstoff oder C₁-C₆-Alkyl bedeuten,
D und E unabhängig voneinander eine Gruppe der Formel 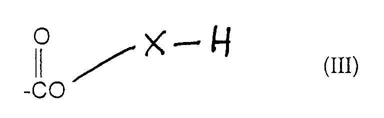 bedeuten und D auch Wasserstoff sein kann, wobei in Formel III
X an dem am Sauerstoff gebundenen Kohlenstoff verzweigtes C₃-C₁₄-Alkylen, C₃-C₈-Alkenylen oder
C₃-C₆-Cycloalkylen-(CH₂)ᵣ- und
r eine Zahl von Null bis 6 bedeuten.

Bedeutet X C₃-C₁₄-Alkylen, so handelt es sich um verzweigtes Alkylen, wie z.B. 1,1-Dimethyl-methylen, 1,1-Dimethyl-dimethylen, 1,1-Dimethyl-trimethylen, 1-Ethyl-dimethylen, 1-Ethyl-1-methyl-dimethylen, 1,1-Dimethyl-tetramethylen, 1,1-Dimethyl-decamethylen oder 1,1-Diethyl-decamethylen.

X als C₃-C₈-Alkenylen bedeutet geradkettiges oder verzweigtes Alkenylen, wie z.B. Methallylen, 1-Methyl-2-butenylen, oder 1,1-Dimethyl-3-butenylen.

Bedeuten etwaige Substitutenten Halogen, dann handelt es sich z.B. um Jod, Fluor, insbesondere Brom und bevorzugt Chlor;
bei C₁-C₆-Alkyl handelt es sich beispielsweise um Methyl, Ethyl, n-Propyl, Isopropyl, n-Butyl, sec.-Butyl, tert.-Butyl, n-Amyl, tert.-Amyl, Hexyl und bei C₁-C₁₈-Alkyl zusätzlich z.B. um Heptyl, Octyl, 2-Ethylhexyl, Nonyl, Decyl, Dodecyl, Tetradecyl, Hexadecyl oder Octadecyl;
C₁-C₄-Alkoxy bedeutet z.B. Methoxy, Ethoxy, n-Propoxy, Isopropoxy, Butyloxy, und C₁-C₁₈-Alkoxy zusätzlich z.B. Hexyloxy, Decyloxy, Dodecyloxy, Hexadecyloxy oder Octadecyloxy;
C₁-C₁₈-Alkylmercapto steht beispielsweise für Methylmercapto, Ethylmercapto, Propylmercapto, Butylmercapto, Octylmercapto, Decylmercapto, Hexadecylmercapto oder Octadecylmercapto;

C₁-C₁₈-Alkylamino bedeutet z.B. Methylamino, Ethylamino, Propylamino, Hexylamino, Decylamino, Hexadecylamino oder Octadecylamino.

C₅-C₆-Cycloalkyl bedeutet z.B. Cyclopentyl und insbesondere Cyclohexyl.

C₃-C₆-Cycloalkylen steht z.B. für Cyclopropylen, Cyclopentylen und insbesondere für Cyclohexylenen.

Von besonderem Interesse sind Pyrrolo[3,4-c]pyrrole der Formel I, worin A und B unabhängig voneinander eine Gruppe der Formel 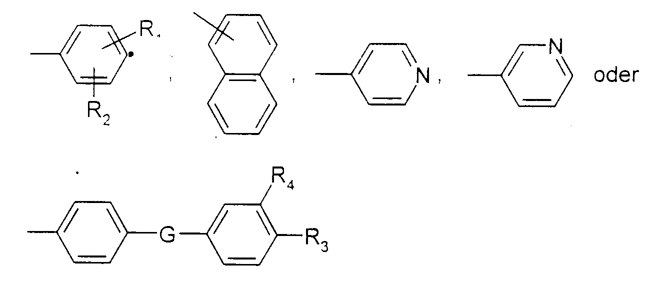 sind,
worin R₁ und R₂ unabhängig voneinander Wasserstoff, Chlor, Brom, C₁-C₄-Alkyl, C₁-C₆-Alkoxy, C₁-C₆-Alkylamino, CN oder Phenyl bedeuten,
G -O-, -NR₇-, -N=N- oder -SO₂- ist, und
R₃ und R₄ Wasserstoff und R₇ Wasserstoff, Methyl oder Ethyl bedeuten, wobei vorzugsweise A und B gleich sind, und insbesondere jene, worin A und B eine Gruppe der Formel 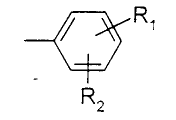 sind, worin R₁ und R₂ unabhängig voneinander Wasserstoff, Methyl, tert.-Butyl, Chlor, Brom, CN oder Phenyl bedeuten. R₂ ist bevorzugt Wasserstoff.

Bevorzugt sind Pyrrolo[3,4-c]pyrrole der Formel I, worin D Wasserstoff bedeutet oder gleich E ist und E eine Gruppe der Formel 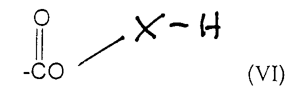 bedeutet, worin
X 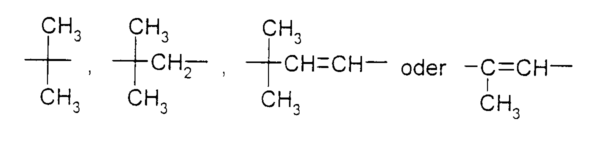 bedeutet
und insbesondere jene, worin D und E gleich sind und eine Gruppe der Formel 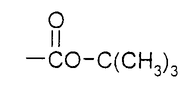 bedeuten.

Dabei ist gemeint, dass bevorzugte Reste D und E auch mit bevorzugten Resten A und B kombiniert werden können.

Zur Herstellung von N-substituierten Pyrrolopyrrolen wird im Patent US-A-4 585 878 beispielsweise erwähnt, dass man sie durch Umsetzung eines N-unsubstituierten Pyrrolo[3,4-c]pyrrols mit einer die entsprechenden N-Substituenten als Abgangsgruppen enthaltenden Verbindung in einem organischen Lösungsmittel, erhalten kann. Im einzigen eine N-Carbonylgruppe enthaltenden Verbindung beschriebenen Beispiel (Beispiel 9: N-Benzoyl) wird 1,4-Diketo-3,6-diphenyl-pyrrolo[3,4-c]pyrrol mit Benzoylchlorid umgesetzt. Beim Versuch, in analoger Weise durch Umsetzung mit einem entsprechenden Säurechloridderivat, z.B. einem Chlorkohlensäureester, die gewünschten Carbamate herzustellen, musste leider festgestellt werden, dass sie nur mit einer schlechten Ausbeute erhalten werden können.

Es wurde aber gefunden, dass beim Verwenden entsprechender Trihaloessigsäureester, Azide, Carbonate, Alkylideniminooxyameisensäureester oder insbesondere entsprechender Dicarbonate, die gewünschten Carbamate ganz überraschend mit sehr guter Ausbeute erhalten werden. Eine wenn auch in geringerem Masse verbesserte Ausbeute wird auch erhalten, wenn die Umsetzung mit einem aliphatischen Säurechloridderivat, z.B. Chlorameisensäure-butylester, in Gegenwart einer Base als Katalysator durchgeführt wird.

Ein weiterer Gegenstand der Erfindung ist demnach ein Verfahren zur Herstellung von Pyrrolo[3,4-c]pyrrolen der Formel 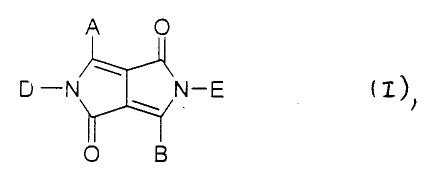 worin A, B, D und E unabhängig voneinander die im Anspruch 1 angegebene Bedeutung aufweisen,
dadurch gekennzeichnet dass ein Pyrrolo[3,4-c]pyrrol der Formel 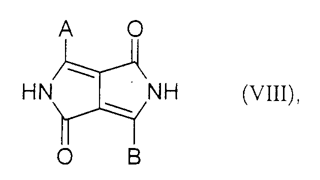 worin A und B die oben angegebene Bedeutung haben,
im gewünschten Molverhältnis mit einem Dicarbonat der Formel

E-O-E (IX)

oder mit einem Trihaloessigsäureester der Formel

(R₁₇)₃C-E (X)

oder mit einem 1:1 Gemisch von Dicarbonat der Formel IX und Dicarbonat der Formel

D-O-D (XI)

oder mit einem 1:1 Gemisch von Trihaloessigsäureester der Formel X und Trihaloessigsäureester der Formel

(R₁₇)₃C-D (XII),

oder mit einem Azid der Formel

E-N₃ (XIII),

welches auch im Gemisch 1:1 mit

D-N₃ (XIV)

eingesetzt werden kann,
oder mit einem Carbonat der Formel

E-OR₁₈ (XV),

welches auch im Gemisch 1:1 mit

D-OR₁₈ (XVI)

eingesetzt werden kann,
worin D und E jeweils die oben angegebene Bedeutung haben, R₁₇ Chlor, Fluor oder Brom, und R₁₈ C₁-C₄-Alkyl oder unsubstituiertes oder durch Halogen, C₁-C₄-Alkyl, C₁-C₄-Alkoxy oder -CN substituiertes Phenyl bedeuten, in einem aprotischen organischen Lösungsmittel in Gegenwart einer Base als Katalysator, umgesetzt wird.

Bevorzugt wird das Pyrrolo[3,4-c]pyrrol der Formel VIII mit einem Dicarbonat der Formel IX oder mit einem 1:1 Gemisch von Dicarbonat der Formel IX und Dicarbonat der Formel XI umgesetzt.

Pyrrolo[3,4-c]pyrrole der Formel VIII, Dicarbonate der Formeln IX und XI, Trihaloessigsäureester der Formeln X und XII, Azide der Formeln XIII und XIV, Carbonate der Formeln XV und XVI und Alkylideniminooxyameisensäureester der Formeln XVII und XVIII sind bekannte Substanzen. Sollten einige noch neu sein, so können sie in Analogie zu allgemein bekannten Methoden hergestellt werden.

Das jeweilige Molverhältnis zwischen Pyrrolo[3,4-c]pyrrol und den Verbindungen der Formeln IX - XVIII richtet sich nach den einzuführenden Resten D und E. Zweckmässig werden die Verbindungen der Formeln IX - XVIII allerdings in 2- bis 10 fachem Ueberschuss eingesetzt.

Als Lösungsmittel eignen sich beispielsweise Ether, wie Tetrahydrofuran oder Dioxan, oder Glykoläther, wie Ethylenglykol-methyläther, Ethylenglykol-ethylether, Diethylenglykol-monomethylether oder Diethylenglykol-monoethylether, ferner dipolar-aprotische Lösungsmittel, wie Acetonitril, Benzonitril, N,N-Dimethylformamid, N,N-Dimethylacetamid, Nitrobenzol, N-Methylpyrrolidon, halogenierte aliphatische oder aromatische Kohlenwasserstoffe, wie Trichlorethan, Benzol oder durch Alkyl, Alkoxy oder Halogen substituiertes Benzol, wie Toluol, Xylol, Anisol oder Chlorbenzol oder aromatische N-Heterocyclen, wie Pyridin, Picolin oder Chinolin. Bevorzugte Lösungsmittel sind z.B. Tetrahydrofuran, N,N-Dimethylformamid, N-Methylpyrrolidon. Die genannten Lösungsmittel können auch als Mischungen eingesetzt werden. Zweckmässigerweise verwendet man 5-20 Gew.-Teile Lösungsmittel auf 1 Gew.-Teil der Reaktionsteilnehmer.

Als Katalysator geeignete Basen sind beispielsweise die Alkalimetalle selbst, wie Lithium-, Natrium- oder Kalium sowie deren Hydroxyde oder Carbonate, oder Alkaliamide, wie Lithium-, Natrium- oder Kaliumamid oder Alkalihydride, wie Lithium-, Natrium- oder Kaliumhydrid, oder Erdalkali- oder Alkalialkoholate, die sich insbesondere von primären, sekundären oder tertiären aliphatischen Alkoholen mit 1 bis 10 C-Atomen ableiten, wie z.B. Lithium-, Natrium- oder Kaliummethylat, -ethylat, -n-propylat, -isopropylat, n-butylat, -sek.-butylat, tert.-butylat, -2-methyl-2-butylat, -2-methyl-2-pentylat, -3-methyl-3-pentylat, -3-ethyl-3-pentylat, und ferner organische aliphatische, aromatische oder heterocyclische N-Basen, darunter z.B. Diazabicycloocten, Diazabicycloundecen und 4-Dimethylaminopyridin und Trialkylamine, wie z.B. Trimethyl- oder Triethylamin. Man kann aber auch ein Gemisch der genannten Basen verwenden.

Bevorzugt werden die organischen N-Basen, wie z.B. Diazabicycloocten, Diazabicycloundecen und insbesondere 4-Dimethylaminopyridin.

Die Umsetzung wird besonders bevorzugt bei Temperaturen zwischen 10 und 100°C, insbesondere zwischen 14 und 40°C, durchgeführt und zwar bei atmosphärischem Druck.

Die erfindungsgemässen Pyrrolopyrrole eignen sich ausgezeichnet als Fluoreszenzfarbstoffe zum Färben von hochmolekularem organischem Material in der Masse.

Beispiele geeigneter hochmolekularer organischer Materialien, die mit den erfindungsgemässen Verbindungen der Formel I gefärbt werden können, sind Vinylpolymere, wie z.B. Polystyrol, Poly-α-methylstyrol, Poly-p-methylstyrol, Poly-p-hydroxystyrol, Poly-p-hydroxyphenylstyrol, Poly(methylacrylat) und Poly(acrylamid) sowie die entsprechenden Methacrylverbindungen, Poly(methylmaleat), Poly(acrylnitril), Poly(methacrylnitril), Poly(vinylchlorid), Poly(vinylfluorid), Poly(vinylidenchlorid), Poly(vinylidenfluorid), Poly(vinylacetat), Poly(methylvinylether) und Poly(butylvinylether); Novolake abgeleitet von C₁-C₆-Aldehyden, wie z.B. Formaldehyd und Acetaldehyd, und einem zweikernigen, vorzugsweise einkernigen Phenol, das gegebenenfalls mit einer oder zwei C₁-C₉-Alkylgruppen, einem oder zwei Halogenatomen oder einem Phenylring substituiert ist, wie beispielsweise o-, m- oder p-Kresol, Xylol, p-tert-Butylphenol, o-, m- oder p-Nonylphenol, p-Chlorphenol oder p-Phenylphenol, oder einer Verbindung mit mehr als einer phenolischen Gruppe, wie z.B. Resorcin, Bis-(4-hydroxyphenyl)methan oder 2,2-Bis-(4-hydroxyphenyl)propan; von Maleinimid und/oder Maleinanhydrid abgeleitete Polymere, wie z.B. Copolymere von Maleinanhydrid und Styrol; Poly(vinylpyrrolidon), Biopolymere und deren Derivate, wie z.B. Cellulose, Stärke, Chitin, Chitosan, Gelatine, Zein, Ethylcellulose, Nitrocellulose, Celluloseacetat und Cellulosebutyrat; natürliche Harze und Kunstharze, wie z.B. Gummi, Casein, Silikon und Silikonharze, ABS, Harnstoff- und Melamin-Formaldehydharze, Alkydharze, Phenolharze, Polyamide, Polyimide, Palyamid/imide, Polysulfone, Polyethersulfone, Polyphenylenoxide, Polyurethane, Polyharhstoffe, Polycarbonate, Polyarylene, Polyarylensulfide, Polyepoxide, Polyolefine und Polyalkadiene. Bevorzugte hochmolekulare organische Materialien, sind z.B. Celluloseether und - ester, wie Ethylcellulose, Nitrocellulose, Celluloseacetat oder Cellulosebutyrat, natürliche Harze oder Kunstharze, wie Polymerisations- oder Kondensationsharze, wie Aminoplaste, insbesondere Harnstoff- und Melamin-Formaldehydharze, Alkydharze, Phenoplaste, Polycarbonate, Polyolefine, Polystyrol, Polyvinylchlorid, Polyamide, Polyurethane, Polyester, ABS, Polyphenylenoxide, Gummi, Casein, Silikon und Silikonharze, einzeln oder in Mischungen.

Die erwähnten hochmolekularen organischen Verbindungen können einzeln oder in Gemischen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemässen Pyrrolopyrrole als Toner oder in Form eines Präparates einzusetzen.

Besonders geeignet sind die erfindungsgemässen Pyrrolopyrrole zum Massefärben von Polyestern, Polyvinylchlorid und insbesondere Polyolefinen, wie Polyethylen und Polypropylen und ABS sowie von Lacken, auch von Pulverlacken, Druckfarben und Anstrichstoffen.

Bezogen auf das zu färbende hochmolekulare organische Material kann man die erfindungsgemässen Pyrrolopyrrole in einer Menge von 0,01 bis 30 Gew.%, vorzugsweise von 0,1 bis 10 Gew. %, einsetzen.

Die Einfärbung der hochmolekularen organischen Substanzen mit den erfindungsgemässen Pyrrolopyrrolen erfolgt beispielsweise derart, dass man das Pyrrolopyrrol, gegebenenfalls in Form von Masterbatches, diesen Substraten unter Verwendung von Walzwerken, Misch- oder Mahlapparaten zumischt. Das gefärbet Material wird hierauf nach an sich bekannten Verfahren, wie Kalandrieren, Pressen, Strangpressen, Streichen, Giessen oder Spritzgiessen, in die gewünschte endgültige Form gebracht. Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können z.B. Ester der Phosphorsäure, Phthalsäure oder Sebacinsäure dienen. Die Weichmacher können vor oder nach der Einverleibung des erfindungsgemässen Pyrrolopyrrols in die Polymeren eingearbeitet werden. Zwecks Erzielung verschiedener Farbtöne ist es ferner möglich, den hochmolekularen organischen Stoffen neben dem erfindungsgemässen Pyrrolopyrrol noch Füllstoffe bzw. andere farbgebende Bestandteile, wie Weiss-, Bunt- oder Schwarzpigmente, in beliebigen Mengen zuzufügen.

Zum Einfärben von Lacken, Anstrichstoffen und Druckfarben werden die hochmolekularen organischen Materialien und die erfindungsgemässen Pyrrolopyrrole gegebenenfalls zusammen mit Zusatzstoffen, wie Füllmitteln, Pigmenten, Siccativen oder Weichmachern, in einem gemeinsamen organischen Lösungsmittel oder Lösungsmittelgemisch fein dispergiert bzw. gelöst. Man kann dabei so verfahren, dass man die einzelnen Komponenten für sich oder auch mehrere gemeinsam dispergiert bzw. löst, und erst hierauf alle Komponenten zusammenbringt.

In Färbungen, beispielsweise von Polyvinylchlorid oder Polyolefinen, zeichnen sich die erfindungsgemässen Pyrrolopyrrole durch gute allgemeine Eigenschaften, wie gute Migrations-, Licht- und Wetterbeständigkeit, aber insbesondere durch die unerwartet hohe Fluoreszenz aus.

Von ganz grosser Bedeutung ist aber die ganz unerwartete Leichtigkeit mit der die erfindungsgemässen Pyrrolopyrrole, selbst im Substrat in welchem sie bereits eingearbeitet wurden, zu den entsprechenden Pyrrolopyrrol-Pigmenten der Formel VIII umgewandelt werden können. Dies kann auf einfachste Weise erzielt werden, sei es durch thermische (Aufheizen auf Temperaturen zwischen 50 und 400°C, bevorzugt zwischen 100 und 200°C oder Laserbestrahlung), photolytische (Belichtung z.B. mit Wellenlängen unter 375 nm) oder chemische (mit organischen oder anorganischen Säuren oder Basen) Behandlung der die erfindungsgemässen Pyrrolopyrrole enthaltenden Festkörper, Lösungen oder Dispersionen in organischen oder wässrigen Medien, Polymerlösungen oder Schmelzen. Die erwähnten Umwandlungsmethoden können auch kombiniert werden. Dies erlaubt die Einfärbung von Lacken, Druckfarben, insbesondere für Inkjet und Kunststoffen, auch z.B. in Faserform, mit ungeahnt verbesserten Eigenschaften, wie Reinheit, Farbstärke, Brillanz und Transparenz, sowie interessante Applikationen in der Analytik.

Ein weiterer Gegenstand der Erfindung betrifft ein Verfahren zum Pigmentieren eines hochmolekularen organischen Materials in der Masse durch thermische, photolitische oder chemische Behandlung eines ein Pyrrolo[3,4-c]pyrrol der Formel I enthaltenden Materials, wobei ein Pyrrolo[3,4-c]pyrrol der Formel 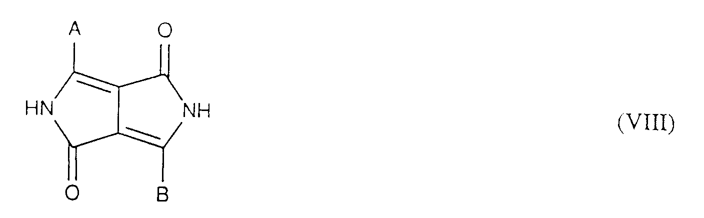 entsteht
worin A und B die für die Formel I angegebene Bedeutung haben, sowie thermo-, photo- oder chemosensitives Aufzeichnungsmaterial und auch photo- und elektrolumineszente Materialien enthaltend ein erfindungsgemässes Pyrrolo[3,4-c]pyrrol der Formel I.

Es ist sogar gefunden worden, dass im Fall bestimmter Pyrrolo[3,4-c]pyrrole der Formel I, die chemische Behandlung mit einer organischen oder anorganischen Säure bei 50 bis 150°C und anschliessende Abkühlung bis ≤ 30°C, oder die thermische Behandlung durch Erhitzen auf Temperaturen von 180 - 350°C zu neuen Kristallmodifikationen der entsprechenden Pyrrolo[3,4-c]pyrrole der Formel VIII führen kann.

Die nachfolgenden Beispiele erläutern die Erfindung.

Beispiel 1: Einer Mischung aus 14,75 g (0,0512 Mol) 1,4-Diketo-3,6-diphenyl-pyrrolo[3,4-c]pyrrol und 3,23 g (0,0264 Mol) 4-Dimethylaminopyridin in 500 ml Tetrahydrofuran (über Molekularsieb getrocknet) werden in 3 Portionen im Abstand von einer Stunde 27,94 g (0,128 Mol) Di-tert.-butyldicarbonat zugegeben. Die erhaltene rote Suspension wird 2 Stunden bei Raumtemperatur unter Ausschluss von atmosphärischer Feuchtigkeit gerührt. Man erhält eine dunkelgrüne Lösung. Das Lösungsmittel wird bei vermindertem Druck abdestilliert. Der gelbe Rückstand wird mit einer 5 %igen wässrigen Natriumbicarbonatlösung gewaschen, mit Wasser ausgespült und im Vakuum getrocknet. Man erhält 24,5 g (98 % d.Th.) des Pyrrolo[3,4-c]pyrrols der Formel

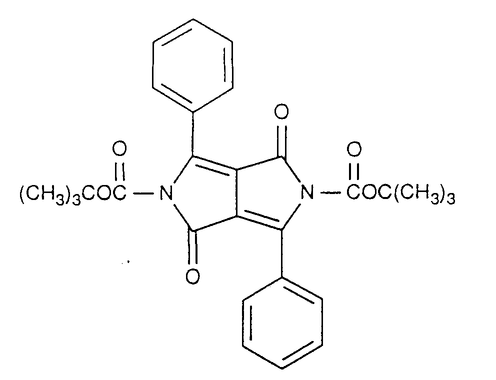

### Analyse:

¹H-NMR (CDCl₃): 7,75 (d, 4H); 7,48-7,50 (m, 6H); 1,40 (s, 18H).

Beispiel 2: Einer Suspension von 4,29 g (0,012 Mol) 1,4-Diketo-3,6-di-(4-chlorphenyl)pyrrolo[3,4-c]pyrrol in 250 ml N,N-Dimethylformamid (über Molekularsieb getrocknet) werden 0,85 g (0,007 Mol) 4-Dimethylaminopyridin und danach 6,55 g (0,030 Mol) Di-tert-butyldicarbonat zugegeben. Das Reaktionsgemisch wird bei Raumtemperatur unter Ausschluss von atmosphärischer Feuchtigkeit gerührt. Nach 2 Stunden werden zusätzliche 6,55 g Di-tert.-butyldicarbonat zugegeben und 72 Stunden weitergerührt. Danach wird die Reaktionsmischung unter kräftigem Rühren in 500 ml destilliertem Wasser gegossen. Die ausgefallene braun-orange Substanz wird abfiltriert, der Rückstand wird mit kaltem destilliertem Wasser gewaschen und im Vakuum bei Raumtemperatur getrocknet. Man erhält 6,1 g (91 % d.Th.) des Pyrrolo[3,4-c]pyrrols der Formel

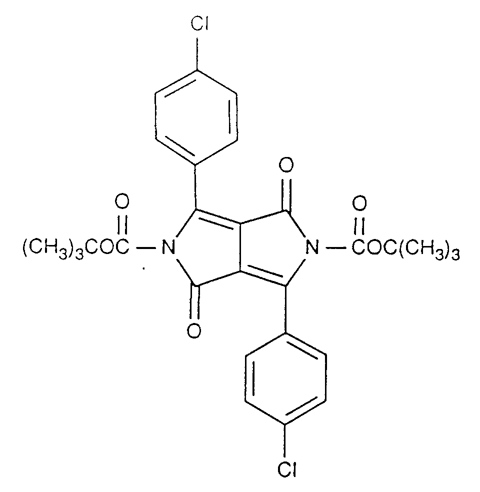

### Analyse:

¹H-NMR (CDCl₃): 7,69 (d, 4H); 7,46 (d, 4H); 1,44 (s, 18H).

Beispiel 3: Einer Mischung aus 8,44 g (0,021 Mol) 1,4-Diketo-3,6-di-(4-tert.-butylphenyl)-pyrrolo[3,4-c]pyrrol und 1,49 g (0,012 Mol) 4-Dimethylaminopyridin in 100 ml N,N-Dimethylformamid (über Molekularsieb getrocknet) werden 24,29 g (0,111 Mol) Di-tert.-butyldicarbonat zugegeben. Die erhaltene rote Suspension wird 3 Stunden bei Raumtemperatur unter Ausschluss von atmosphärischer Feuchtigkeit gerührt. Es erfolgt ein Farbumschlag nach Orange. Die ausgefallene Substanz wird abfiltriert, der Rückstand wird wiederholt mit kaltem destilliertem Wasser gewaschen und im Vakuum bei Raumtemperatur getrocknet. Man erhält 11,40 g (90 % d.Th.) eines leuchtend gelben Produktes der Formel

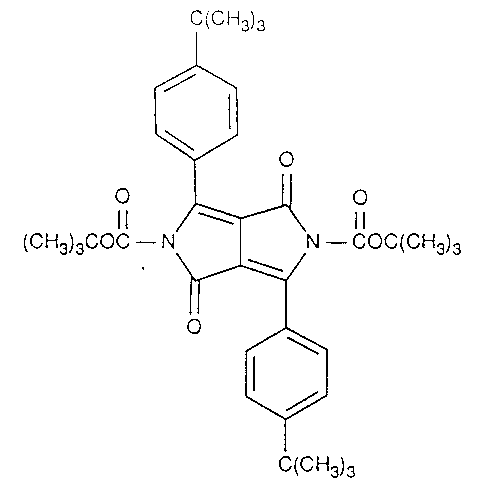

### Analyse:

¹H-NMR (CDCl₃): 7,69 (d, 4H); 7,48 (d, 4H); 1,43 (s, 18H); 1,34 (s, 18H).

Beispiele 4-12: In analoger Weise können bei Verwendung von Pyrrolo[3,4-c]pyrrolen der Formel 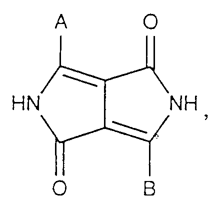 worin A und B die in der nachstehenden Tabelle in den Kolonnen 2 und 3 angegebene Bedeutung haben, entsprechende erfindungsgemässe Pyrrolo[3,4-c]pyrrole der Formel 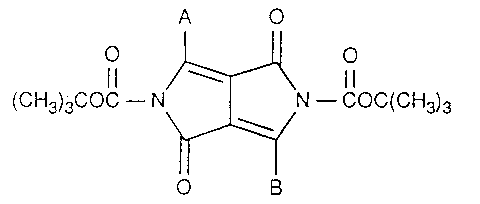 hergestellt werden. In der nachstehenden Tabelle sind in Kolonne 4 die Lösungsmittel, in Kolonne 5 die Reaktionszeit, in Kolonne 6 die Ausbeute und in Kolonne 7 die Analysenwerte ¹H-NMR (CDCl₃) angegeben.

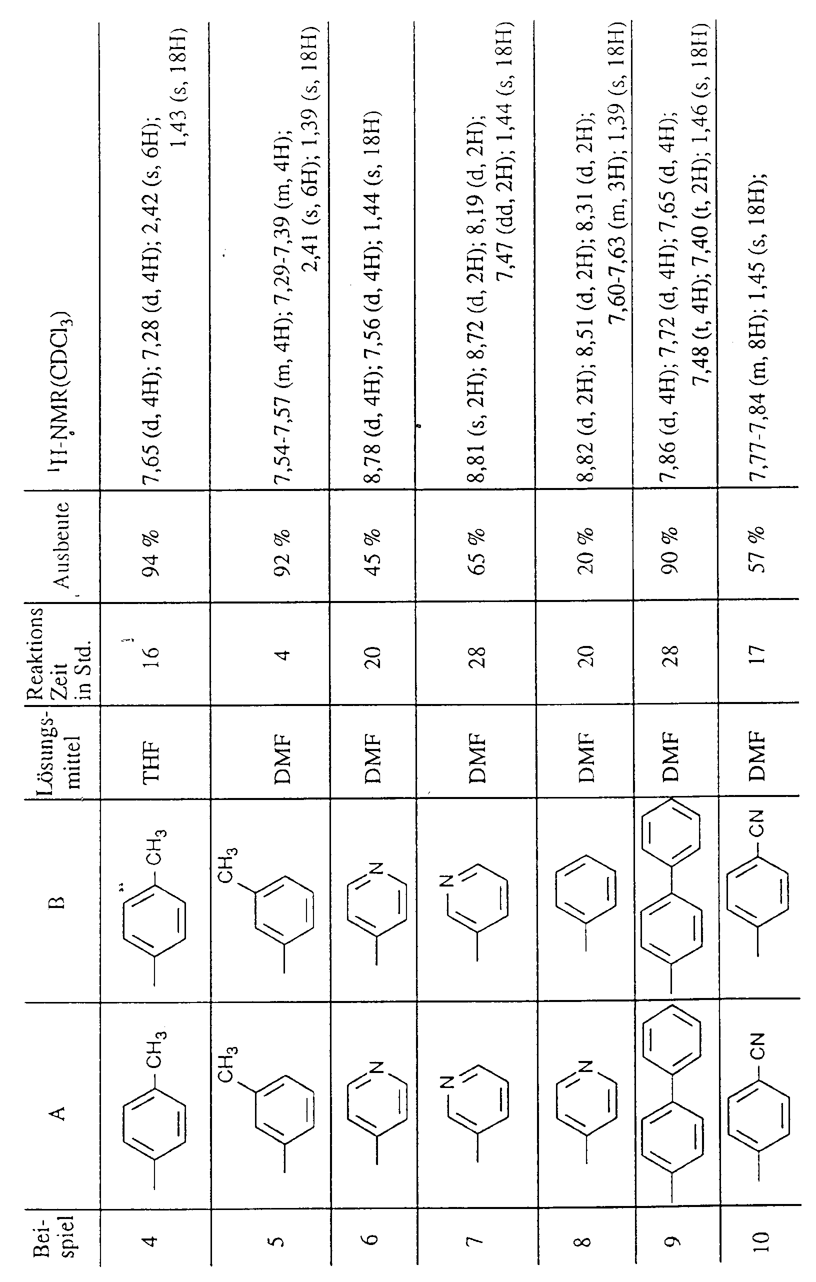

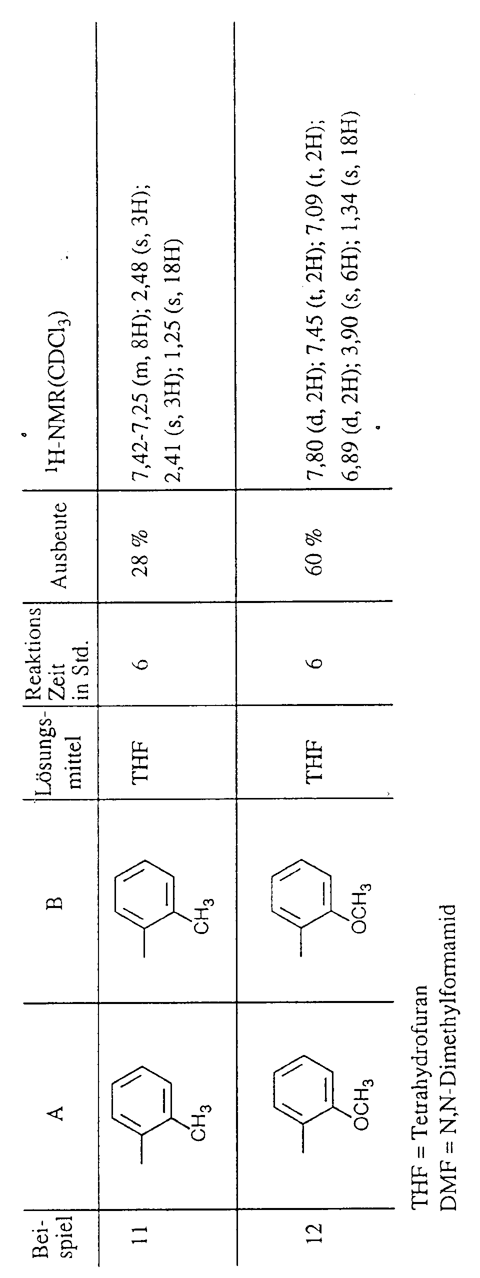

Beispiele 13-15: In Analoger Weise wie in Beispiel 1 können, bei Verwendung entsprechender Dicarbonate, die in der nachstehenden Tabelle angeführten Verbindungen der Formel 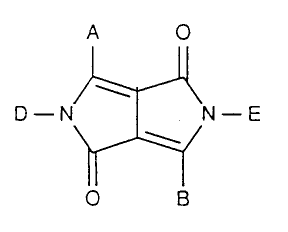 erhalten werden.

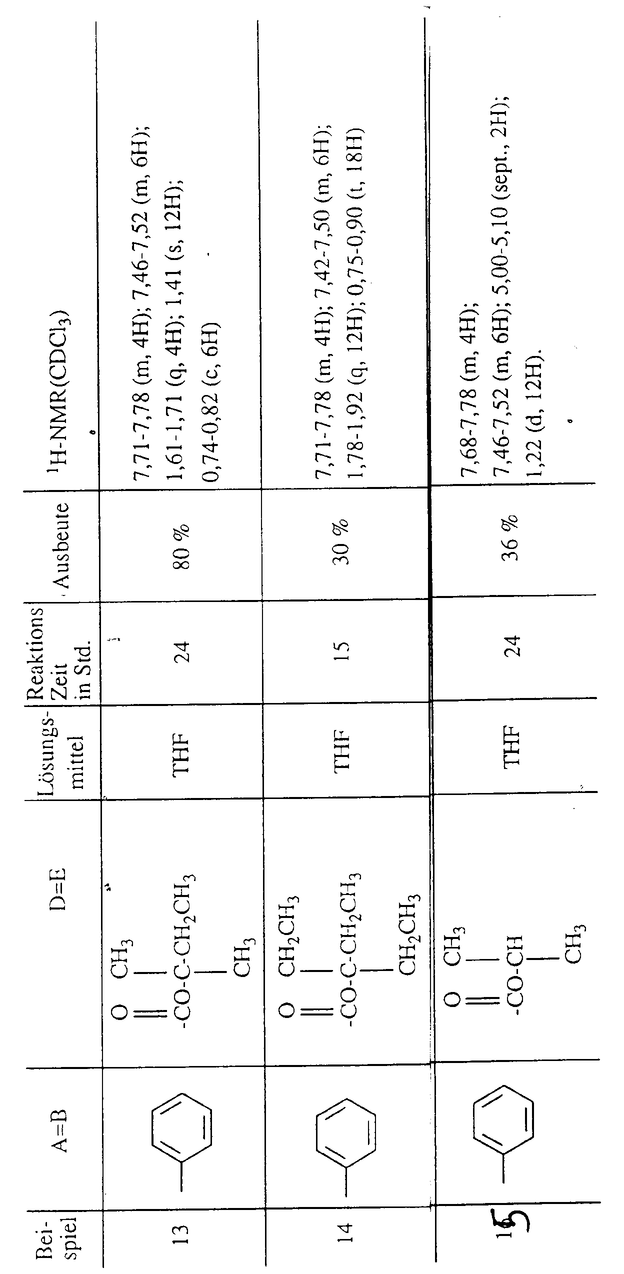

Beispiel 16: 14,93 g N,N'-Bis(tert.-butoxycarbonyl)-1,4-diketo-3,6-diphenyl-pyrrolo[3,4-c]pyrrol, hergestellt, wie in Beispiel 1 beschrieben, werden aus 1,1 l kochendem Ethanol umkristallisert. Die ausgefallenen roten Kristalle werden über einer Kieselgel-Säule mit Methylenchlorid/Ethylacetat 9:1 als Laufmittel chromatographiert. Man erhält das Diketopyrrolo[3,4-c]pyrrol der Formel

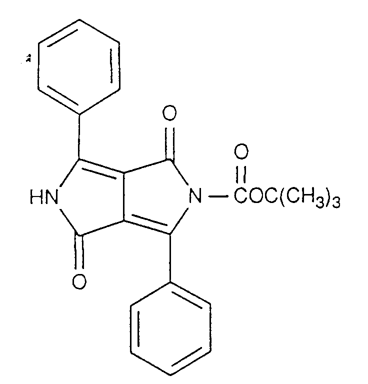

### Analyse:

¹H-NMR (CDCl₃): 9,43 (s, br, 1H); 8,30 (m, 2H); 7,81 (m, 2H); 7,51 (m, 6H); 1,4 (s, 9H).

Beispiele 17-21: In analoger Weise können aus den entsprechenden disubstituierten Pyrrolo[3,4-c]pyrrolen die in der nachstehenden Tabelle angeführten monosubstituierten Verbindungen der Formel 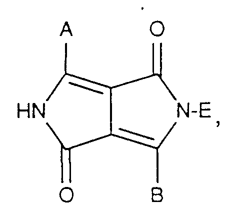 erhalten werden.

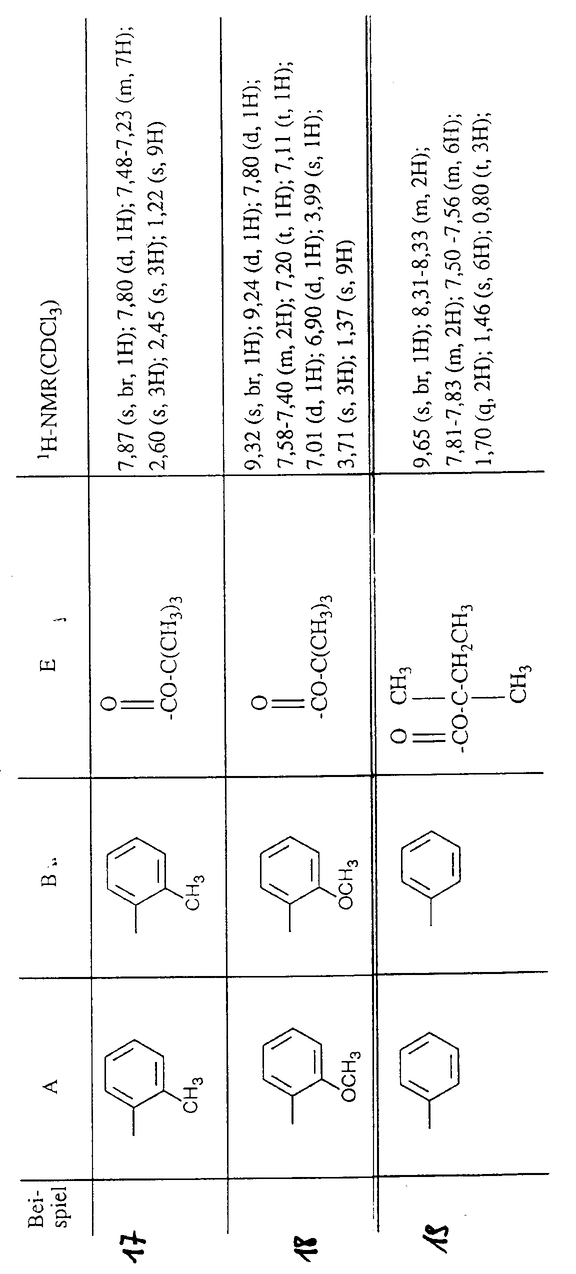

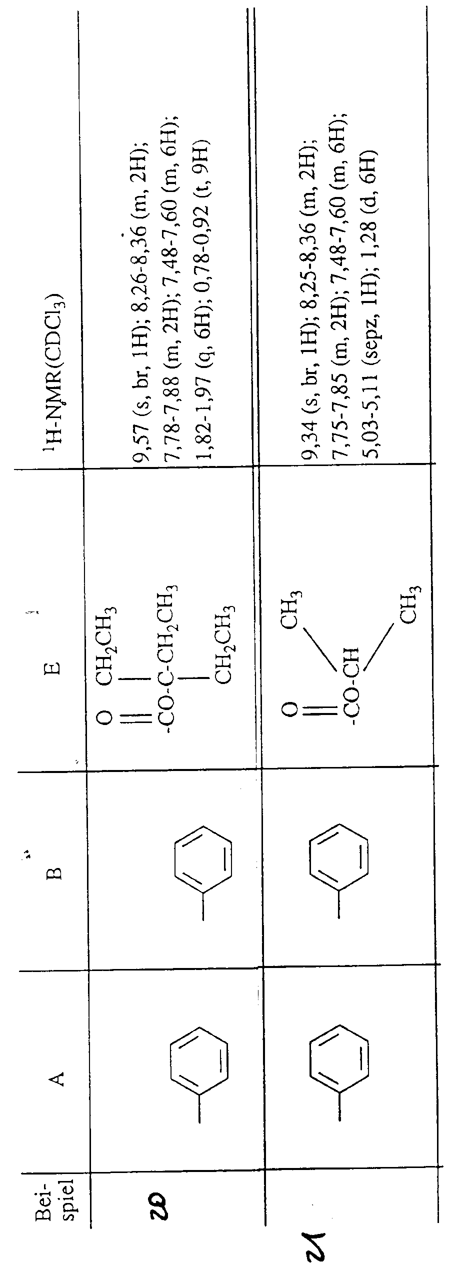

Beispiel 22: 0,07 g N,N-Bis(tert.-butoxycarbonyl)-1,4-diketo-3,6-diphenyl-pyrrolo[3,4-c]pyrrol aus Beispiel 1 werden in einem Reagenzglas 10 Minuten bei 180°C erhitzt. Alle analytischen Daten des entstehenden roten Pulvers entsprechen denjenigen von reinem 1,4-Diketo-3,6-diphenyl-pyrrolo[3,4-c]pyrrol. Die Ausbeute der Umwandlung beträgt 99 %.

Beispiel 23: 0,07 g N,N-Bis(tert.-butoxycarbonyl)-1,4-diketo-3,6-diphenyl-pyrrolo[3,4-c]pyrrol aus Beispiel 1 werden in 1 ml Aceton gelöst und anschliessend auf einmal 1 ml 33 %ige HCl zugegeben. Alle analytischen Daten des ausgefallenen roten Pulvers entsprechen denjenigen von reinem 1,4-Diketo-3,6-diphenyl-pyrrolo[3,4-c]pyrrol. Die Ausbeute der Umwandlung beträgt 99 %.

## Patentansprüche

1. Pyrrolo[3,4-c]pyrrole der Formel 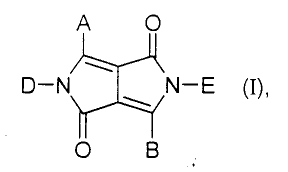 worin A und B unabhängig voneinander für eine Gruppe der Formel 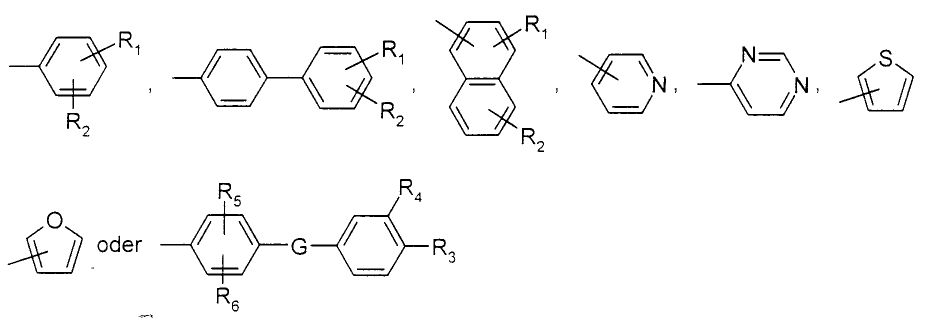 stehen, worin
R₁ und R₂ unabhängig voneinander Wasserstoff, Halogen, C₁-C₁₈-Alkyl, C₁-C₁₈-Alkoxy, C₁-C₁₈-Alkylmercapto, C₁-C₁₈-Alkylamino, -CN, -NO₂, -Phenyl, Trifluormethyl, C₅-C₆-Cycloalkyl,
-C=N-(C₁-C₁₈-Alkyl), 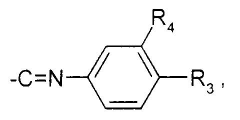 Imidazolyl, Pyrrazolyl, Triazolyl, Piperazinyl,
Pyrrolyl, Oxazolyl, Benzoxazolyl, Benzthiazolyl, Benzimidazolyl, Morpholinyl, Piperidinyl oder Pyrrolidinyl bedeuten,
G -CH₂-, -CH(CH₃)-, -C(CH₃)₂-, -CH=N-, -N=N-, -O-, -S-,-SO-, -SO₂- oder -NR₇- ist,
R₃ und R₄ unabhängig voneinander Wasserstoff, Halogen, C₁-C₆-Alkyl, C₁-C₁₈Alkoxy oder -CN sind, R₅ und R₆ unabhängig voneinander Wasserstoff, Halogen oder C₁-C₆-Alkyl und R₇ Wasserstoff oder C₁-C₆-Alkyl bedeuten,
D und E unabhängig voneinander eine Gruppe der Formel 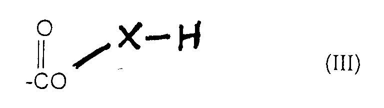 bedeuten und D auch Wasserstoff sein kann, wobei in Formel III
X an dem am Sauerstoff gebundenen Kohlenstoff verzweigtes C₃-C₁₄-Alkylen, C₃-C₈-Alkenylen oder
C₃-C₆-Cycloalkylen-(CH₂)ᵣ-, und
r eine Zahl von Null bis 6 bedeuten.

2. Pyrrolo[3,4-c]pyrrole gemäss Anspruch 1, dadurch gekennzeichnet, dass in der Formel I A und B unabhängig voneinander eine Gruppe der Formel 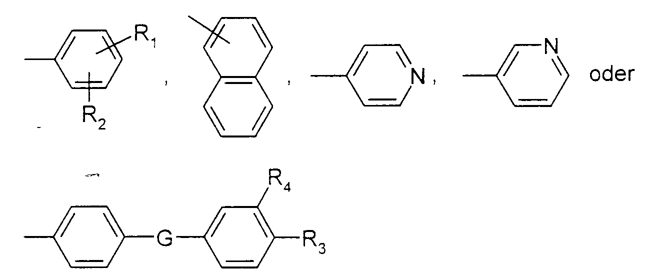 sind, worin R₁ und R₂ unabhängig voneinander Wasserstoff, Chlor, Brom, C₁-C₄-Alkyl, C₁-C₆-Alkoxy, C₁-C₆-Alkylamino, CN oder Phenyl bedeuten,
G -O-, -NR₇-, -N=N- oder -SO₂- ist, und
R₃ und R₄ Wasserstoff und R₇ Wasserstoff, Methyl oder Ethyl bedeuten.

3. Pyrrolo[3,4-c]pyrrole gemäss Anspruch 1, dadurch gekennzeichnet, dass in der Formel I A und B gleich sind.

4. Pyrrolo[3,4-c]pyrrole gemäss Anspruch 3, dadurch gekennzeichnet, dass in der Formel I A und B eine Gruppe der Formel 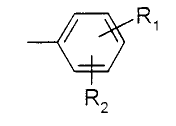 sind, worin R₁ und R₂ unabhängig voneinander Wasserstoff, Methyl, tert.-Butyl, Chlor, Brom, CN oder Phenyl bedeuten.

5. Pyrrolo[3,4-c]pyrrole gemäss Anspruch 1, dadurch gekennzeichnet, dass in der Formel I D Wasserstoff bedeutet oder gleich E ist und E eine Gruppe der Formel 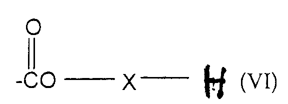 bedeutet, worin
X 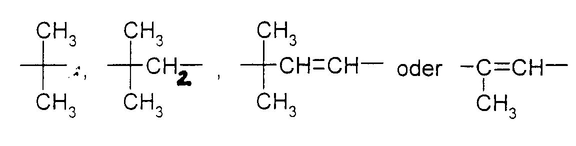 bedeutet.

6. Pyrrolo[3,4-c]pyrrole gemäss Anspruch 1, dadurch gekennzeichnet, dass in der Formel I D und E gleich sind und eine Gruppe der Formel 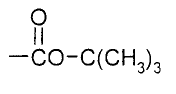 bedeuten.

7. Verfahren zur Herstellung von Pyrrolo[3,4-c]pyrrolen der Formel 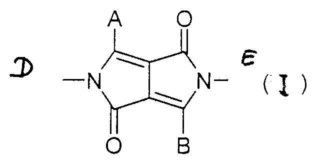 gemäß
Anspruch 1
dadurch gekennzeichnet dass ein Pyrrolo[3,4-c]pyrrol der Formel 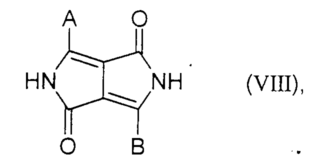 worin A und B die in Anspruch 1 angegebene Bedeutung haben,
im gewünschten Molverhältnis mit einem Dicarbonat der Formel
E-O-E (IX)
oder mit einem Trihaloessigsäureester der Formel
(R₁₇)₃C-E (X)
oder mit einem 1:1 Gemisch von Dicarbonat der Formel IX und Dicarbonat der Formel
D-O-D (XI)
oder mit einem 1:1 Gemisch von Trihaloessigsäureester der Formel X und Trihaloessigsäureester der Formel
(R₁₇)₃C-D (XII),
oder mit einem Azid der Formel
E-N₃ (XIII),
welches auch im Gemisch 1:1 mit
D-N₃ (XIV)
eingesetzt werden kann,
oder mit einem Carbonat der Formel
E-OR₁₈ (XV),
welches auch im Gemisch 1:1 mit
D-OR₁₈ (XVI)
eingesetzt werden kann,
worin D und E jeweils die in Anspruch 1 angegebene Bedeutung haben, R₁₇ Chlor, Fluor oder Brom, und R₁₈ C₁-C₄-Alkyl oder unsubstituiertes oder durch Halogen, C₁-C₄-Alkyl, C₁-C₄-Alkoxy oder -CN substituiertes Phenyl bedeuten, in einem aprotischen organischen Lösungsmittel in Gegenwart einer Base als Katalysator, umgesetzt wird.

8. Verfahren gemäss Anspruch 7, dadurch gekennzeichnet, dass das Pyrrolo[3,4-c]pyrrol der Formel VII mit einem Dicarbonat der Formel IX oder mit einem 1:1 Gemisch von Dicarbonat der Formel IX und Dicarbonat der Formel XI umgesetzt wird.

9. Hochmolekulares organisches Material enthaltend in der Masse ein Pyrrolo[3,4-c]pyrrol gemäss Anspruch 1 als Fluoreszenzfarbstoff.

10. Thermo-, photo- oder chemosensitives Aufzeichnungsmaterial enthaltend ein Pyrrolo[3,4-c]pyrrol der Formel I gemäss Anspruch 1.

11. Photo- und elektrolumineszente Materialien enthaltend ein Pyrrolo[3,4-c]pyrrol der Formel I gemäss Anspruch 1.

12. Verfahren zum Pigmentieren eines hochmolekularen organischen Materials, dadurch gekennzeichnet, dass
(1) ein Pyrrolo[3,4-c]pyrrol der Formel 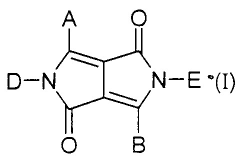 gemäss Anspruch 1 in das hochmolekulare organische Material eingearbeitet wird, und dann
(2) das im hochmolekularen organischen Material eingearbeitete Pyrrolo[3,4-c]pyrrol der Formel (I) durch thermische, photolytische oder chemische Behandlung in ein Pyrrolo[3,4-c]pyrrol der Formel 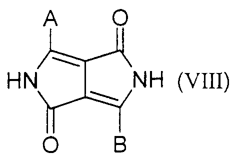 umgewamdelt wird, worin A und B die gleiche Bedeutung haben, wie in Formel (I).

## Claims

1. A pyrrolo[3,4-c]pyrrole of formula 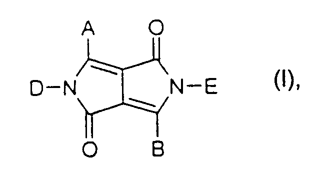 wherein A and B are each independently of the other a group of formula 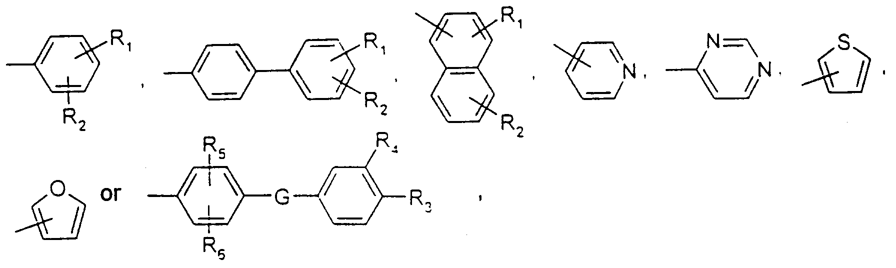 wherein R₁ and R₂ are each independently of the other hydrogen, halogen, C₁-C₁₈alkyl, C₁-C₁₈alkoxy, C₁-C₁₈alkylmercapto, C₁-C₁₈alkylamino, -CN, -NO₂, phenyl, trifluoromethyl, C₅-C₆cycloalkyl, -C=N-(C₁-C₁₈alkyl), 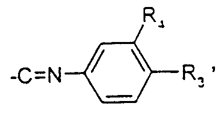 imidazolyl, pyrrazolyl, triazolyl, piperazinyl, pyrrolyl, oxazolyl, benzoxazolyl, benzothiazolyl, benzimidazolyl, morpholinyl, piperidinyl or pyrrolidinyl,
G is -CH₂-, -CH(CH₃)-, -C(CH₃)₂-, -CH=N-, -N=N-, -O-, -S-, -SO-, -SO₂ or -NR₇-,
R₃ and R₄ are each independently of the other hydrogen, halogen, C₁-C₆alkyl, C₁-C₁₈alkoxy or -CN, R₅ and R₆ are each independently of the other hydrogen, halogen or C₁-C₆alkyl and R₇ is hydrogen or C₁-C₆alkyl,
D and E are each independently of the other a group of formula 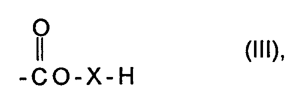 and D may also be hydrogen, and in formula III
X is C₃-C₁₄alkylene, C₃-C₈alkenylene, each branched at the carbon bonded to the oxygen, or C₃-C₆cycloalkylene-(CH₂)ᵣ-, and
r is an integer from 0 to 6.

2. A pyrrolo[3,4-c]pyrrole according to claim 1, wherein A and B in formula I are each independently of the other a group of formula 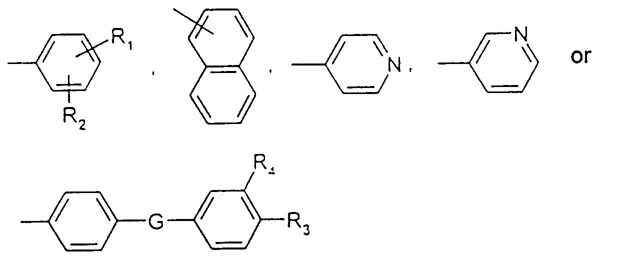 wherein R₁ and R₂ are each independently of the other hydrogen, chloro, bromo, C₁-C₄alkyl, C₁-C₆alkoxy, C₁-C₆alkylamino, CN or phenyl,
G is -O-, -NR₇-, -N=N- or -SO₂-, and
R₃ and R₄ are hydrogen and R₇ is hydrogen, methyl or ethyl.

3. A pyrrolo[3,4-c]pyrrole according to claim 1, wherein A and B in formula I are identical.

4. A pyrrolo[3,4-c]pyrrole according to claim 3, wherein A and B in formula I are a group of formula 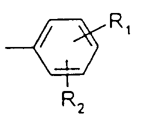 wherein R₁ and R₂ are each independently of the other hydrogen, methyl, tert-butyl, chloro, bromo, CN or phenyl.

5. A pyrrolo[3,4-c]pyrrole according to claim 1, wherein D in formula I is hydrogen or is identical to E and E is a group of formula 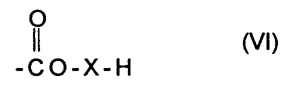 wherein
X is

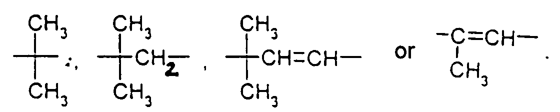

6. A pyrrolo[3,4-c]pyrrole according to claim 1, wherein D and E in formula I are identical and are a group of formula

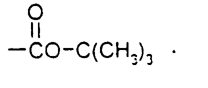

7. A process for the preparation of a pyrrolo[3,4-c]pyrrole of formula 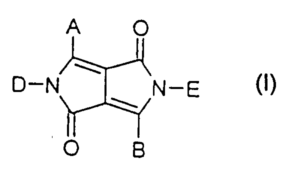 according to claim 1,
which process comprises reacting a pyrrolo[3,4-c]pyrrole of formula 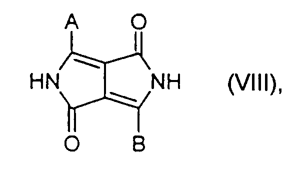 wherein A and B are as defined in claim 1,
in the desired molar ratio with a dicarbonate of formula
E-O-E (IX),
or with a trihaloacetate of formula
(R₁₇)₃C-E (X),
or with a 1:1 mixture of a dicarbonate of formula IX and a dicarbonate of formula
D-O-D (XI),
or with a 1:1 mixture of a trihaloacetate of formula X and a trihaloacetate of formula
(R₁₇)₃C-D (XII),
or with an azide of formula
E-N₃ (XIII),
which may also be used in a 1:1 mixture with
D-N₃ (XIV),
or with a carbonate of formula
E-OR₁₈ (XV),
which may also be used in a 1:1 mixture with
D-OR₁₈ (XVI),
wherein D and E are each as defined in claim 1, R₁₇ is chloro, fluoro or bromo, and R₁₈ is C₁-C₄alkyl or unsubstituted phenyl or phenyl which is substituted by halogen, C₁-C₄alkyl, C₁-C₄alkoxy or -CN, in an aprotic organic solvent in the presence of a base as catalyst.

8. A process according to claim 7, which comprises reacting the pyrrolo[3,4-c]pyrrole of formula VIII with a dicarbonate of formula IX or with a 1:1 mixture of a dicarbonate of formula IX and a dicarbonate of formula XI.

9. High molecular weight organic material comprising in the mass a pyrrolo[3,4-c]pyrrole according to claim 1 as fluorescent dye.

10. Heat-sensitive, photosensitive or chemosensitive recording material comprising a pyrrolo[3,4-c]pyrrole of formula I according to claim 1.

11. Photo- and electro-luminescent material comprising a pyrrolo[3,4-c]pyrrole of formula I according to claim 1.

12. A process for the pigmenting of a high molecular weight organic material, which process comprises
(1) incorporating a pyrrolo[3,4-c]pyrrole of formula 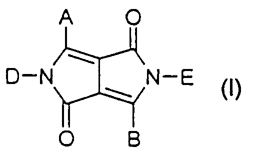 according to claim 1 into the high molecular weight organic material and then,
(2) by thermal, photolytic or chemical treatment, converting the pyrrolo[3,4-c]pyrrole of formula (I) incorporated in the high molecular weight organic material into a pyrrolo[3,4-c]pyrrole of formula 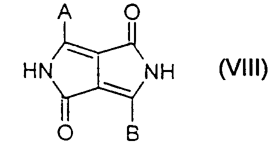 wherein A and B are as defined for formula (I).

## Revendications

1. Pyrrolo[3,4-c]pyrrole de formule 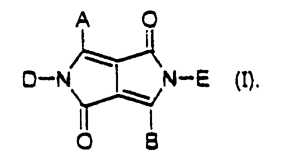 dans laquelle A et B représentent indépendamment l'un de l'autre un groupe de formule 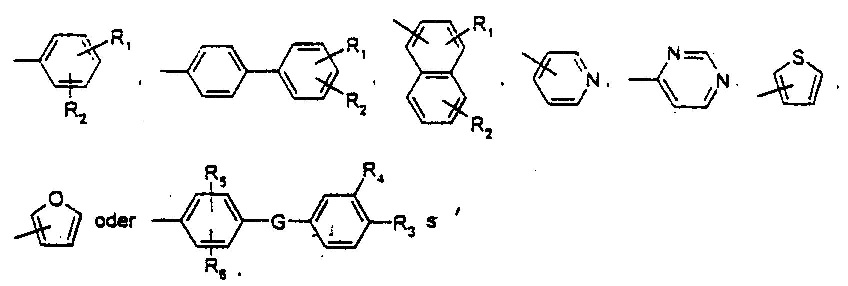 dans laquelle
R₁ et R₂ représentent indépendamment l'un de l'autre un atome d'hydrogène, d'halogène, un groupe alkyle en C₁ à C₁₈, alcoxy en C₁ à C₁₈, alkylmercapto en C₁ à C₁₈, alkylamino en C₁ à C₁₈, -CN, -NO₂, -phényle, trifluorométhyle, cycloalkyle en C₅ à C₆, C=N-(alkyle en C₁ à C₁₈), 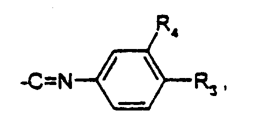 imidazolyle, pyrrazolyle, triazolyle, pipérazinyle, pyrrolyle, oxazolyle, benzoxazolyle, benzthiazolyle, benzimidazolyle, morpholinyle, pipéridinyle ou pyrrolidinyle.
G représente un groupe -CH₂-, -CH(CH₃)-, -C(CH₃)₂-, -CH=N-, -N=N, -O-, -S-, -SO-, -SO₂ ou -NR₇,
R₃ et R₄ représentent indépendamment l'un de l'autre un atome d'hydrogène, d'halogène, un groupe alkyle en C₁ à C₆, alcoxy en C₁ à C₁₈ ou -CN,
R₅ et R₆ représentent indépendamment l'un de l'autre un atome d'hydrogène, d'halogène ou un groupe alkyle en C₁ à C₆, et
R₇ représente un atome d'hydrogène ou un groupe alkyle en C₁ à C₆,
D et E représentent indépendamment l'un de l'autre un groupe de formule 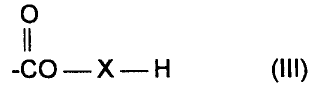 et D peut être aussi un atome d'hydrogène, dans la formule III
X représente un groupe alkylène en en C₃ à C₁₄ ramifié dont un atome de carbone est lié à l'oxygène, alcénylène en C₃ à C₈ ou cycloalkylène en C₃ à C₈-(CH₂)ᵣ- et
r représente un nombre de zéro à 6.

2. Pyrrolo[3,4-c]pyrrole selon la revendication 1, caractérisé en ce que dans la formule I A et B représentent indépendamment l'un de l'autre un groupe de formule 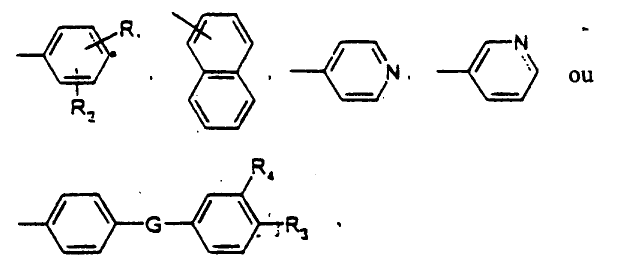 dans lesquelles
R₁ et R₂ représentent indépendamment l'un de l'autre un atome d'hydrogène, de chlore, de brome, un groupe alkyle en C₁ à C₄, alcoxy en C₁ à C₆, alkylamino en C₁ à C₆, CN ou phényle.
G représente -O-, -NR₇, -N=N- ou -SO₂-, et
R₃ et R₄ représentent un atome d'hydrogène et
R₇ représente un atome d'hydrogène, un groupe méthyle ou éthyle.

3. Pyrrolo[3,4-c]pyrrole selon la revendication 1, caractérisé en ce que dans la formule I, A et B sont identiques.

4. Pyrrolo[3,4-c]pyrrole selon la revendication 1, caractérisé en ce que dans la formule I, A et B sont un groupe de formule 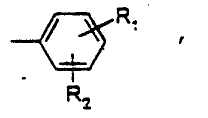 dans laquelle R₁ et R₂ représentent indépendamment l'un de l'autre un atome d'hydrogène, un groupe méthyle, tert.-butyle, un atome de chlore, de brome, un groupe CN ou phényle.

5. Pyrrolo[3,4-c]pyrrole selon la revendication 1, caractérisé en ce que dans la formule I, D représente un atome d'hydrogène ou est identique à E et E représente un groupe de formule 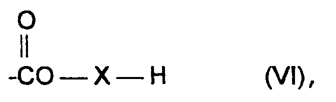 dans laquelle
X représente

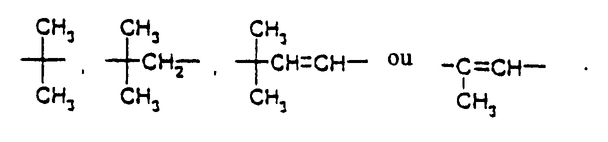

6. Pyrrolo[3,4-c]pyrrole selon la revendication 1, caractérisé en ce que dans la formule I, D et E sont identiques et représentent un groupe de formule

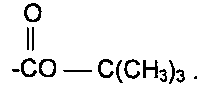

7. Procédé pour la préparation de pyrrolo[3,4-c]pyrroles de formule 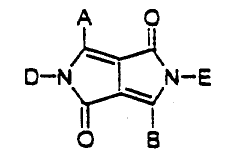 selon la revendication 1, caractérisé en ce qu'on met à réagir un pyrrolo[3,4-c]pyrrole de formule 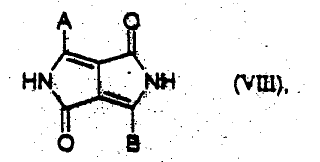 dans laquelle A et B ont la signification indiquée à la revendication 1,
dans le rapport molaire souhaité avec un dicarbonate de formule
E-O-E (IX)
ou avec un trihalogénoacétate de formule
(R₁₇)₃C-E (X),
ou avec un mélange 1:1 de dicarbonate de formule IX et de dicarbonate de formule
D-O-D (XI)
ou avec un mélange 1:1 de trihalogénoacétate de formule X et de trihalogénoacétate de formule
(R₁₇)₃C-D (XII),
ou avec un azide de formule
E―N₃ (XIII),
qui peut aussi être utilisé en mélange 1:1 avec
D―N₃ (XIV),
ou avec un carbonate de formule
E-OR₁₈ (XV),
qui peut aussi être utilisé en mélange 1:1 avec
D-OR₁₈ (XVI).
formules dans lesquelles D et E on respectivement les significations indiquées à la revendication 1, R₁₇ représente un atome de chlore, de fluor ou de brome, et R₁₈ représente un groupe alkyle en C₁ à C₄ ou un groupe phényle non substitué ou substitué par un atome d'halogène, un groupe alkyle en C₁ à C₄, alcoxy en C₁ à C₄ ou -CN, dans un solvant organique aprotique en présence d'une base comme catalyseur.

8. Procédé selon la revendication 7, caractérisé en ce qu'on met à réagir le pyrrolo[3,4-c]pyrrole de formule VII avec un dicarbonate de formule IX ou avec un mélange 1:1 de dicarbonate de formule IX et de dicarbonate de formule XI.

9. Matériau organique de masse moléculaire élevée contenant dans la masse un pyrrolo[3,4-c]pyrrole selon la revendication 1 comme colorant de fluorescence.

10. Matériau d'enregistrement thermo-, photo- ou chimiosensible contenant un pyrrolo[3,4-c]pyrrole de formule I selon la revendication 1.

11. Matériaux photo- et électroluminescents contenant un pyrrolo[3,4-c[pyrrole de formule I selon la revendication 1.

12. Procédé pour la pigmentation d'un matériau organique de masse moléculaire élevée, caractérisé en ce que
(1) l'on incorpore un pyrrolo[3,4-c]pyrrole de formule 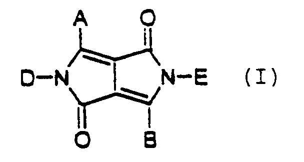 selon la revendication 1 dans le matériau organique de masse moléculaire élevée et en ce qu'ensuite
(2) l'on transforme le pyrrolo[3,4-c]pyrrole de formule (I) incorporé dans le matériau organique de masse moléculaire élevée par traitement thermique, photolytique ou chimique en un pyrrolo[3,4-c]pyrrole de formule 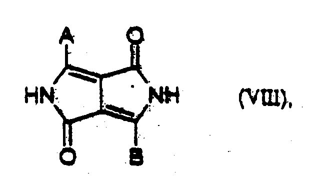 dans laquelle A et B ont la même signification que dans la formule (I).
